# EUROPEAN PATENT APPLICATION

(11) **EP 2 803 661 A1**
(43) Date of publication of application: **19.11.2014**
(21) Application number: 13168051.4
(22) Date of filing: 16.05.2013
(51) Int. Cl.: C07D 213/803, C07C 67/343, C07D 309/32, C07C 69/738

(54) **Method for preparation of 6-trifluoromethylpyridine-3-carboxylic acid derivatives**

(71) Applicant: Lonza Ltd, 3930 Visp (CH)
(72) Inventor: Zaragoza Dörwald, Florencio, 3930 Visp (CH); Gantenbein, Annabelle, 3930 Visp (CH); Taeschler, Christoph, 3930 Visp (CH); Bersier, Michael, 3938 Ausserberg (CH)

(57) **Abstract**

The invention discloses a method for the preparation of substituted 6-trifluoromethylpyridine-3-carboxylic acid derivatives starting from 1,1,1-trifluoroacetone or from synthetic equivalents of 1,1,1-trifluoroacetone.

## Description

The invention discloses a method for the preparation of substituted 6-trifluoromethylpyridine-3-carboxylic acid derivatives starting from 1,1,1-trifluoroacetone or from synthetic equivalents of 1,1,1-trifluoroacetone.

### BACKGROUND OF THE INVENTION

2-Trifluoromethylpyridines and 6-trifluoromethylpyridine-3-carboxylic acid derivatives are intermediates for the preparation of biologically active compounds. For instance, WO 00/39094 A1 discloses trifluoromethylpyridine as herbicides, WO 2006/059103 A2 discloses trifluoromethylpyridines as intermediates in the production of pharmaceutical, chemical and agro-chemical products, WO 2008/013414 A1 discloses trifluoromethylpyridines as vanilloid receptor antagonists and WO 2012/061926 A1 describes trifluoromethylpyridines as calcium channel blockers.

The common route for the preparation of 6-trifluoromethylpyridine-3-carboxylic acid derivatives was first reported by Okada et al., Heterocycles 1997, 46, 129-132, and has only been slightly modified by others. The common synthetic strategies are summarized in Scheme 1:

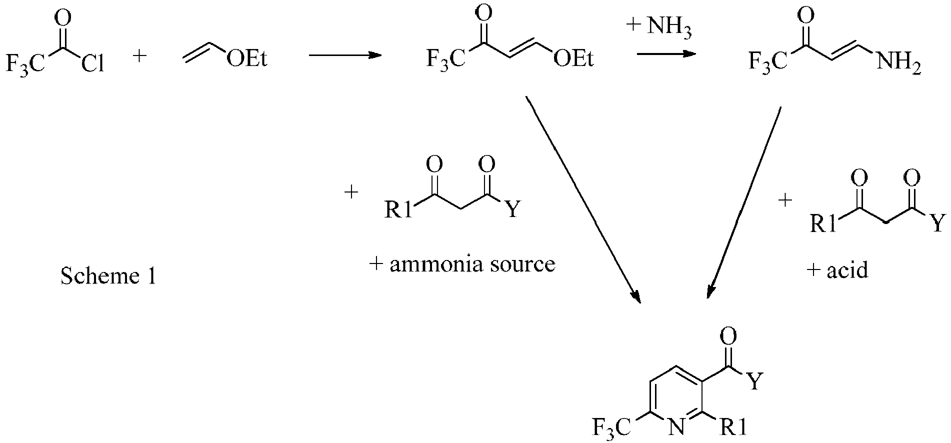

This route has disadvantages for the large scale production of 6-trifluoromethylpyridine-3-carboxylic acid derivatives, because ethylvinylether is highly flammable and therefore difficult to handle, and because the trifluoroacetylated enolether and the trifluoroacetylated enamine intermediates are unstable and cannot be stored for a longer time. Moreover, most vinyl ethers are mutagenic.

There was a need for an improved procedure for the preparation of 6-trifluoromethylpyridine-3-carboxylic acid derivatives.

This need was met by the method of instant invention as outlined below.

Compared to prior art, the method of the instant invention offers several advantages: Importantly, no vinyl ethers, trifluoroacetylated enolether intermediates or trifluoroacetylated enamine intermediates are required. Moreover, the method of the present invention only comprises few synthetic steps, what reduces the overall costs.

In the following text, if not otherwise stated,
- halogen: means F, Cl, Br or I, preferably Cl, Br or I;
- alkyl: means a linear or branched alkyl, examples of alkyl include methyl, ethyl, n-propyl, isopropyl, n-butyl, sec-butyl, tert-butyl, pentyl, hexyl, heptyl and the like; cyclic alkyl or cyclo alkyl include cyclo aliphatic, bicyclo aliphatic and tricycle aliphatic residues; examples of "cycloalkyl" include cyclopropyl, cyclobutyl, cyclopentyl, cyclohexyl, cycloheptyl, norbornyl and adamantyl;
- alkoxy: means alkyl-O, i.e. the radical obtained by removal of the oxygen-bound hydrogen from an aliphatic alcohol; (alkoxy)alkoxy refers to alkoxy groups, in which the alkyl group is substituted with one additional alkoxy group; examples of (alkoxy)alkoxy include methoxymethoxy with formula MeO-CH₂-O-, 2-(methoxy)ethoxy with formula MeO-CH₂-CH₂-O- and 2-(cyclopropylmethoxy)ethoxy with formula (C₃H₅)CH₂-O-CH₂-CH₂-O-;
- Ac: acetyl;
- tBu: tertiary butyl;
- DBU: 1,8-diazabicyclo[5.4.0]undec-7-ene;
- DABCO: 1,4-diazabicyclo[2.2.2]octane;
- DMF: N,N-dimethylformamide;
- DMA: N,N-dimethylacetamide;
- DMSO: dimethylsulfoxide;
- halogen: means F, Cl, Br or J, preferably F, Cl or Br;
- hemiacetal: refers to the adduct of an alcohol, for instance methanol or ethanol, with a ketone or with an aldehyde; a hemiacetal may also result upon the addition of water to an enol ether; for instance, the hemiacetal of methanol with trifluoroacetone is F₃C-C(OH)(OCH₃)-CH₃;
- hexanes: mixture of isomeric hexanes;
- hydrate: refers to the adduct of water with a ketone or with an aldehyde, for instance, the hydrate of trifluoroacetone is F₃C-C(OH)₂-CH₃;
- LDA: Lithium diisopropyl amide
- NMP: N-methyl-2-pyrrolidone;
- sulfamic acid: HO-SO₂-NH₂;
- THF: tetrahydrofuran;
- trifluoroacetone: 1,1,1-trifluoropropan-2-one;
- xylene: 1,2-dimethylbenzene, 1,3-dimethylbenzene, 1,4-dimethylbenzene or a mixture thereof.

### SUMMARY OF THE INVENTION

Subject of the invention is a method for the preparation of compound of formula (II); compound of formula (II) is selected from the group consisting of compound of formula (IIa), compound of formula (IIb), compound of formula (IIc), a hydrate or a hemiacetal of compound of formula (IIa), of compound of formula (IIb) and of compound of formula (IIc) with a C₁₋₄ alkanol, and mixtures thereof; 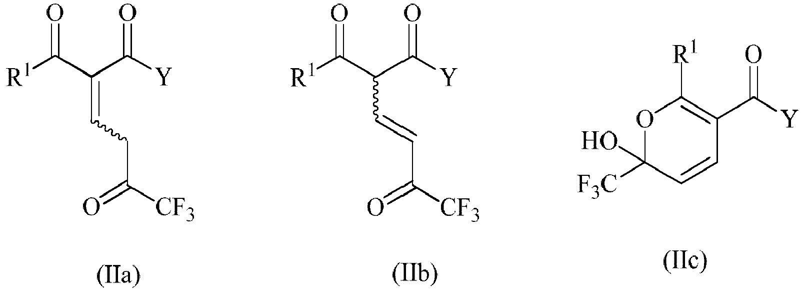 wherein the method comprises a step (StepS1);
step (StepS1) comprises a reaction (ReacS1);
reaction (ReacS1) is a reaction of a compound of formula (III) with a compound (TFK) in the presence of a base (BasS1); 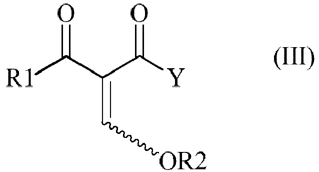 compound (TFK) is 1,1,1-trifluoroacetone or a synthetic equivalent of 1,1,1-trifluoroacetone;
base (BasS1) is selected from the group consisting of sodium hydride, potassium hydride, calcium hydride, sodium amide, lithium amide, LDA, N-lithium-2,2,6,6-tetramethylpiperidide, N-magnesium-2,2,6,6-tetramethylpiperidide, sodium C₁₋₄ alkoxide, potassium C₁₋₄ alkoxide, sodium carbonate, potassium carbonate, C₁₋₄ alkyl magnesium bromide and C₁₋₄ alkyl magnesium chloride;
- R1: is selected from the group consisting of C₁₋₁₀ alkyl, C₃₋₈ cycloalkyl, C(O)-O-C₁₋₄ alkyl, CH=CH₂, benzyl, phenyl and naphthyl;
the C₁₋₁₀ alkyl of R1 is unsubstituted or substituted with 1, 2, 3, 4 or 5 identical or different substituents selected from the group consisting of halogen, OH, O-C(O)-C₁₋₅ alkyl, O-C₁₋₁₀ alkyl, S-C₁₋₁₀ alkyl, S(O)-C₁₋₁₀ alkyl, S(O₂)-C₁₋₁₀ alkyl, O-C₁₋₆ alkylen-O-C₁₋₆ alkyl, C₃₋₈ cycloalkyl and 1,2,4-triazolyl;
the benzyl, the phenyl and the naphthyl of R1 are independently from each other unsubstituted or substituted with 1, 2, 3, 4 or 5 identical or different substituents selected from the group consisting of halogen, C₁₋₄ alkoxy, NO₂ and CN;
- R2: is selected from the group consisting of C₁₋₄ alkyl;
- Y: is selected from the group consisting of OH, C₁₋₆ alkoxy, O-C₁₋₆ alkylen-O-C₁₋₆ alkyl, NH₂, NHR4 and N(R4)R5;
- R4 and R5: are identical or different and independently from each other C₁₋₆ alkyl.

### DETAILED DESCRIPTION OF THE INVENTION

Preferably, a hydrate or a hemiacetal of compound of formula (IIa), of compound of formula (IIb) and of compound of formula (IIc) with a C₁₋₄ alkanol is a hydrate or a hemiacetal of compound of formula (IIa), of compound of formula (IIb) and of compound of formula (IIc) with MeOH or with EtOH.

Preferably, the synthetic equivalent of 1,1,1-trifluoroacetone is selected from the group consisting of compound of formula (IV), 4,4,4-trifluoro-3-oxobutyric acid and a salt of 4,4,4-trifluoro-3-oxobutyric acid; 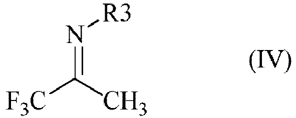 wherein
- R3: is selected from the group consisting of hydrogen, C₁₋₆ alkyl, benzyl, phenyl, pivaloyl, methanesulfonyl, benzenesulfonyl, 4-methylbenzenesulfonyl, C(O)-C₁₋₄ alkyl, O-C₁₋₁₀ alkyl, O-benzyl, NH₂, NHR6 and N(R6)R7;
- R6 and R7: are identical or different and have independently from each other and from R4 the same definition as R4.

Preferably, the salt of 4,4,4-trifluoro-3-oxobutyric acid is selected from the group consisting of Na⁺, K⁺, NH₄⁺ and Ca²⁺ salt of 4,4,4-trifluoro-3-oxobutyric acid.

Preferably, base (BasS1) is selected from the group consisting of sodium hydride, potassium hydride, calcium hydride, sodium amide, lithium amide, LDA, N-lithium-2,2,6,6-tetramethylpiperidide, N-magnesium-2,2,6,6-tetramethylpiperidide, sodium methoxide, sodium ethoxide, sodium isopropoxide, sodium tert-butoxide, potassium tert-butoxide sodium carbonate, potassium carbonate, isopropylmagnesium bromide, isopropylmagnesium chloride, and mixtures thereof;
more preferably, base (BasS1) is selected from the group consisting of sodium hydride, potassium hydride, calcium hydride, sodium amide, lithium amide, LDA, sodium methoxide, sodium ethoxide, sodium isopropoxide, sodium tert-butoxide, potassium tert-butoxide, sodium carbonate, potassium carbonate, and mixtures thereof;
even more preferably, base (BasS1) is selected from the group consisting of sodium hydride, sodium amide, lithium amide, sodium methoxide, sodium ethoxide, sodium isopropoxide, sodium tert-butoxide, potassium tert-butoxide, sodium carbonate, potassium carbonate, and mixtures thereof.

Preferably, R1 is selected from the group consisting of C₁₋₅ alkyl, C₃₋₆ cycloalkyl, C(O)-O-C₁₋₄ alkyl, CH=CH₂, benzyl and phenyl;
the C₁₋₁₀ alkyl of R1 is unsubstituted or substituted with 1, 2, 3, 4 or 5 identical of different substituents selected from the group consisting of halogen, OH, O-C(O)-CH₃, O-C₁₋₅ alkyl, S-C₁₋₅ alkyl, S(O)-C₁₋₅ alkyl, S(O₂)-C₁₋₅ alkyl, O-C₁₋₄ alkylen-O-C₁₋₄ alkyl, C₃₋₆ cycloalkyl and 1,2,4-triazolyl;
the benzyl and the phenyl of R1 are independently from each other unsubstituted or substituted with 1, 2, 3, 4 or 5 identical of different substituents selected from the group consisting of halogen, C₁₋₂ alkoxy, NO₂ and CN;

More preferably, R1 is selected from the group consisting of methyl, ethyl, isopropyl, tert-butyl, cyclopropyl, trifluoromethyl, difluoromethyl, chloromethyl, bromomethyl, C(O)-O-CH₃, C(O)-O-C₂H₅, CH₂-O-C(O)-CH₃, CH₂- O-CH₃, CH₂-S-CH₃, CH₂-S(O₂)-CH₃, CH₂-CH₂- O-CH₃, CH₂-O-CH₂-CH₂-O-CH₃, CH₂-O-CH₂-CH₂-O-CH₂-CH₃, CH=CH₂ and phenyl.

Preferably, Y is selected from the group consisting of OH, methoxy and ethoxy.

Preferably, R2 is selected from the group consisting of methyl and ethyl.

Preferably, R3 is selected from the group consisting of hydrogen, C₁₋₆ alkyl, benzyl, phenyl, pivaloyl, methanesulfonyl, benzenesulfonyl, 4-methylbenzenesulfonyl, methoxycarbonyl, ethoxycarbonyl, O-C₁₋₄ alkyl, O-benzyl, NH₂ and dimethylamino;
more preferably, R3 is selected from the group consisting of hydrogen, C₁₋₆ alkyl, benzyl, phenyl, pivaloyl, methoxycarbonyl, ethoxycarbonyl, methoxy, ethoxy, NH₂ and dimethylamino.

Preferably, reaction (ReacS1) is done in a solvent (SolvS1);
solvent (SolvS1) is selected from the group consisting of THF, 2-methyl-THF, 3-methyl-THF, DMF, DMA, acetonitrile, propionitrile, DMSO, 1,2-dimethoxyethane, methoxycyclopentane, diethyleneglycoldimethylether, diethyleneglycoldiethylether, benzene, toluene, anisole, and mixtures thereof.

Preferably, solvent (SolvS1) is selected from the group consisting of THF, 2-methyl-THF, 3-methyl-THF, DMF, DMA, 1,2-dimethoxyethane, benzene, toluene, anisole, and mixtures thereof;
more preferably, solvent (SolvS1) is selected from the group consisting of THF, 2-methyl-THF, 3-methyl-THF, DMF, 1,2-dimethoxyethane, toluene, and mixtures thereof.

Preferably, the weight of solvent (SolvS1) is from 0.1 to 100 times, more preferably from 1 to 50 times, even more preferably from 1 to 10 times of the weight of compound of formula (III).

Preferably, the molar ratio [base (BasS1) : compound of formula (III)] is from [0.1 : 1] to [10 : 1], more preferably from [0.3 : 1] to [5 : 1], even more preferably from [0.5 : 1] to [4 : 1].

Preferably, the molar ratio [compound (TFK) : compound of formula (III)] is from [1 : 1] to [10 : 1], more preferably [1 : 1] to [5 : 1], even more preferably [1 : 1] to [3 : 1] .

Preferably, reaction (ReacS1) is done at a pressure of from 1 to 20 bar, more preferably of from 1 to 15 bar, even more preferably of from 1 to 10 bar.

Preferably, the reaction time of reaction (ReacS1) is from 30 min to 96 h, more preferably from 1 h to 48 h, even more preferably from 2 h to 24 h.

Preferably, the reaction temperature of reaction (ReacS1) is from -70 to 150°C, more preferably from -50 to 120°C, even more preferably from -40 to 100°C.

Preferably, reaction (ReacS1) is done by the addition of compound (TFK) to a mixture of base (BasS1) with solvent (SolvS1) resulting in a mixture (MixS11);
then compound of formula (III) is added to the mixture (MixS11) resulting in a mixture (MixS12).

Preferably, the addition of compound (TFK) to the mixture of base (BasS1) with solvent (SolvS1) is done at a temperature (TempS11);
the mixture (MixS11) is stirred for the time (TimeS11) at the temperature (TempS11);
the addition of compound of formula (III) to the mixture (MixS11) is done at the temperature (TempS11);
the mixture (MixS12) is stirred for the time (TimeS12) at a temperature (TempS12);
after the time (TimeS12) the mixture (MixS12) is stirred for a time (TimeS13) at a temperature (TempS13);
after the time (TimeS13) the mixture (MixS12) is stirred for a time (TimeS14) at a temperature (TempS14); and
preferably, temperature (TempS11) is from -50 to 20°C, more preferably from -40 to 10°C, even more preferably from -30 to 5°C;
preferably, time (TimeS11) is from 1 min to 5 h, more preferably from 10 min to 3 h, even more preferably from 10 min to 2 h;
preferably, temperature (TempS12) is from -30 to 60°C, more preferably from -15 to 40°C, even more preferably from -5 to 30°C;
preferably, time (TimeS 12) is from 10 min to 24 h, more preferably from 30 min to 12 h, even more preferably from 1 h to 6 h;
preferably, temperature (TempS13) is from 0 to 120°C, more preferably from 10 to 100°C, even more preferably from 20 to 90°C;
preferably, time (TimeS13) is from 10 min to 24 h, more preferably from 30 min to 12 h, even more preferably from 1 h to 6 h;
preferably, temperature (TempS14) is from 0 to 120°C, more preferably from 10 to 100°C, even more preferably from 20 to 90°C;
preferably, time (TimeS14) is from 10 min to 24 h, more preferably from 30 min to 12 h, even more preferably from 1 h to 6 h.

Preferably, compound of formula (II) is isolated after reaction (ReacS1) by hydrolysis and acidification of the reaction mixture resulting from reaction (ReacS1). Hydrolysis and acidification is preferably done by addition of a compound (InAcS1), compound (InAcS1) is an aqueous inorganic acid, preferably compound (InAcS1) is selected from the group consisting of aqueous hydrochloric acid and aqueous sulfuric acid. After hydrolysis and acidification, solvent (SolvS1) is preferably removed by distillation, followed by extraction with a solvent (SolvExtrS1), solvent (SolvExtrS1) is preferably selected from the group consisting of ethyl acetate, isopropyl acetate, butyl acetate, toluene, chlorobenzene, dichloromethane, chloroform and mixtures thereof; the extraction is preferably followed by removal of solvent (SolvExtrS1) by distillation.

Compounds of formula (III) are known compounds and can be prepared according or in analogy to known methods, for instance as described by Coates and Hobbs, J. Org. Chem., 1984, 49, 140-152.

Further subject of the invention is a method for the preparation of compound of formula (I); 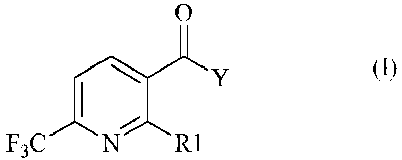 the method comprises the step (StepS1) and a step (StepS2);
with step (StepS1) as defined above, also with all its preferred embodiments;
step (StepS2) comprises a reaction (ReacS2);
reaction (ReacS2) is a reaction of the compound of formula (II) prepared in step (StepS1) with a compound (AMS);
with compound of formula (II), R1 and Y as defined above, also with all their preferred embodiments;
compound (AMS) is selected from the group consisting of NH₃, sulfamic acid, urea, NH₂-C(O)-C₁₋₄ alkyl, ammonium carbonate, ammonium bicarbonate, ammonium salts of C₁₋₄ carboxylic acids, ammonium chloride and mixtures thereof.

Preferably, compound (AMS) is selected from the group consisting of NH₃, formamide, ammonium carbonate, ammonium bicarbonate, ammonium formate, ammonium acetate, ammonium chloride and mixtures thereof.

Preferably, reaction (ReacS2) is done in a solvent (SolvS2); solvent (SolveS2) is selected from the group of C₁₋₆ alkanol, acetonitrile, propionitrile, C₁₋₄ carboxylic acid, benzene, toluene, xylene, chlorobenzene, anisole, pyridine, 2-methylpyridine, 3-methylpyridine, 4-methylpyridine, 2-methyl-5-ethylpyridine, DMF, DMA, DMSO, water, ethyl acetate, acetone, methylethylketone, THF, 2-methyl-THF, 3-methyl-THF, 1,2-dimethoxyethane, diethyleneglycoldimethylether and mixtures thereof;
more preferably, solvent (SolveS2) is selected from the group consisting of C₁₋₆ alkanol, acetonitrile, propionitrile, C₁₋₃ carboxylic acid, toluene, xylene, chlorobenzene, 3-methylpyridine, 2-methyl-5-ethylpyridine, DMF, DMA, water, methylethylketone and mixtures thereof.

Preferably, the weight of solvent (SolvS2) is from 0.1 to 100 times, more preferably from 1 to 50 times, even more preferably from 1 to 10 times of the weight of compound of formula (II).

Preferably, reaction (ReacS2) is done at a temperature from 0 to 200°C, more preferably from 10 to 150°C, even more preferably from 20 to 130°C.

Preferably, reaction (ReacS2) is done at a pressure of from 0.5 to 20 bar, more preferably from 1 to 10 bar, even more preferably from 1 to 5 bar.

Preferably, reaction time of reaction (ReacS2) is from 10 min to 72 h, more preferably from 1 h to 48 h, even more preferably from 2 h to 24 h.

After reaction (ReacS2), compound of formula (I) may be isolated by any conventional method. Preferably, any solvent is distilled off, the residue is diluted with water, the pH is adjusted to 0 to 12, preferably to 2 to 10, more preferably to 3 to 8, by addition of an acid or a base, and compound of formula (I) is either isolated by filtration or extracted with a solvent such as ethyl acetate, isopropyl acetate, butyl acetate, toluene, chlorobenzene, dichloromethane or chloroform; after distilling off the solvent used for the extraction, the crude product may be further purified by recrystallization.

Further subj ect of the invention is the use of compound of formula (II), with compound of formula (II) having been prepared according to a respective method as described above, also with all its preferred embodiments, or of compound of formula (I), with compound of formula (I) having been prepared according to a respective method as described above, also with all its preferred embodiments, for the production of pharmaceutical, chemical or agro-chemical products.

### EXAMPLES

### Example 1: Compound of formula (II) with R1 and Y being methyl

Sodium hydride (60% w/w in oil, 139 mg, 3.5 mmol) was washed with hexane and then suspended in THF (1.4 ml). At 0°C 1,1,1-trifluoroacetone (0.24 ml, 2.7 mmol) was added dropwise to this suspension. The mixture was stirred at 0 °C for 15 min, and then compound of formula (3-1)

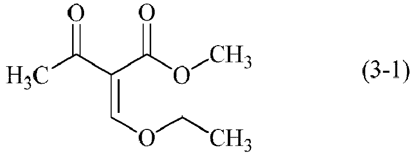

(1.8 mmol, prepared according to Coates and Hobbs, J. Org. Chem. 1984, 49, 140-152) and more THF (0.25 ml) were added. The mixture was stirred at 0°C for 30 min, then at room temperature for 1.5 h, then at 60°C for 2 h. The mixture was then added to aqueous IN HCl (4 ml), and extracted with AcOEt (once with 5 ml and twice with 3 ml). The combined organic extracts were washed with brine, dried with MgSO₄ and concentrated under reduced pressure providing the product as a residual oil (519 mg).
¹H NMR (CDCl₃, 400 MHz, main component): delta 2.45 (s, 3H), 3.79 (s, 3H), 5.56 (d, J = 8 Hz, 1H), 6.94 ppm (d, J = 8 Hz, 1H).
¹⁹F NMR (CDCl3, 376 MHz): delta 84.87 ppm.

The NMR spectra identify the product in the residual oil as a compound of formula (II) with R1 and Y being methyl.

### Example 2: Methyl 2-methyl-6-(trifluoromethyl) nicotinate

To the residual oil (519 mg) prepared according to example 1 were added acetic acid (1.5 ml) and ammonium acetate (525 mg, 6.81 mmol), and the resulting mixture was stirred at 60°C for 16 h. The mixture was added to brine (5 ml), followed by extraction with AcOEt (3 times with 3 ml). The combined extracts were washed with brine, dried with MgSO₄ and concentrated under reduced pressure, to yield an oil. Analysis and quantification by ¹H NMR with internal standard, which was 4-nitro benzaldehyde, indicated a yield of the title compound of 50% with respect to the amount of compound of formula (3-1) used in example 1.
¹H NMR (CDCl₃, 400 MHz): delta 2.89 (s, 3H), 3.97 (s, 3H), 7.60 (d, J = 8 Hz, 1H), 8.35 (d, J = 8 Hz, 1H)
¹⁹F NMR (CDCl3, 376 MHz): delta 68.4

## Claims

1. Method for the preparation of compound of formula (II);
compound of formula (II) is selected from the group consisting of compound of formula (IIa), compound of formula (IIb), compound of formula (IIc), a hydrate or a hemiacetal of compound of formula (IIa), of compound of formula (IIb) and of compound of formula (IIc) with a C₁₋₄ alkanol, and mixtures thereof; 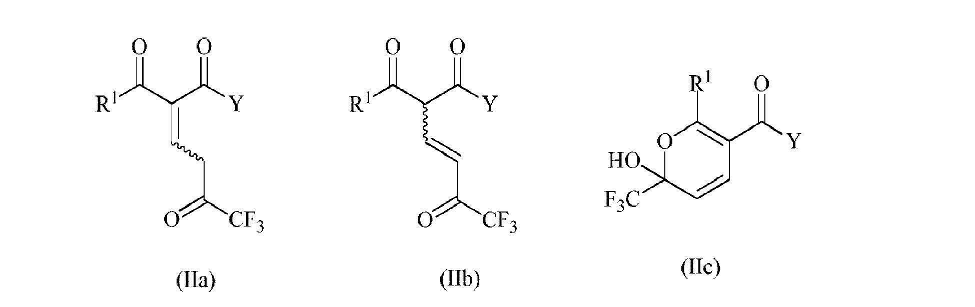 wherein the method comprises a step (StepS1);
step (StepS1) comprises a reaction (ReacS1);
reaction (ReacS1) is a reaction of a compound of formula (III) with a compound (TFK) in the presence of a base (basS1); 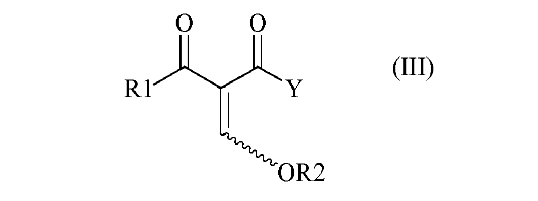 compound (TFK) is 1,1,1-trifluoroacetone or a synthetic equivalent of 1,1,1-trifluoroacetone;
base (BasS1) is selected from the group consisting of sodium hydride, potassium hydride, calcium hydride, sodium amide, lithium amide, LDA, N-lithium-2,2,6,6-tetramethylpiperidide, N-magnesium-2,2,6,6-tetramethylpiperidide, sodium C₁₋₄ alkoxide, potassium C₁₋₄ alkoxide, sodium carbonate, potassium carbonate, C₁₋₄ alkyl magnesium bromide and C₁₋₄ alkyl magnesium chloride;
R1 is selected from the group consisting of C₁₋₁₀ alkyl, C₃₋₈ cycloalkyl, C(O)-O-C₁₋₄ alkyl, CH=CH₂, benzyl, phenyl and naphthyl;
the C₁₋₁₀ alkyl of R1 is unsubstituted or substituted with 1, 2, 3, 4 or 5 identical or different substituents selected from the group consisting of halogen, OH, O-C(O)-C₁₋₅ alkyl, O-C₁₋₁₀ alkyl, S-C₁₋₁₀ alkyl, S(O)-C₁₋₁₀ alkyl, S(O₂)-C₁₋₁₀ alkyl, O-C₁₋₆ alkylen-O-C₁₋₆ alkyl, C₃₋₈ cycloalkyl and 1,2,4-triazolyl;
the benzyl, the phenyl and the naphthyl of R1 are independently from each other unsubstituted or substituted with 1, 2, 3, 4 or 5 identical or different substituents selected from the group consisting of halogen, C₁₋₄ alkoxy, NO₂ and CN;
R2 is selected from the group consisting of C₁₋₄ alkyl;
Y is selected from the group consisting of OH, C₁₋₆ alkoxy, O-C₁₋₆ alkylen-O-C₁₋₆ alkyl, NH₂, NHR4 and N(R4)R5;
R4 and R5 are identical or different and independently from each other C₁₋₆ alkyl.

2. Method according to claim 1, wherein
a hydrate or a hemiacetal of compound of formula (IIa), of compound of formula (IIb) and of compound of formula (IIc) with a C₁₋₄ alkanol is a hydrate or a hemiacetal of compound of formula (IIa), of compound of formula (IIb) and of compound of formula (IIc) with MeOH or with EtOH.

3. Method according to claim 1 or 2, wherein
the synthetic equivalent of 1,1,1-trifluoroacetone is selected from the group consisting of compound of formula (IV), 4,4,4-trifluoro-3-oxobutyric acid and a salt of 4,4,4-trifluoro-3-oxobutyric acid; 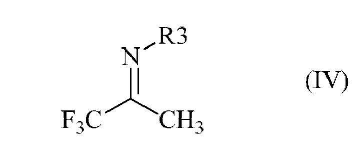 wherein
R3 is selected from the group consisting of hydrogen, C₁₋₆ alkyl, benzyl, phenyl, pivaloyl, methanesulfonyl, benzenesulfonyl, 4-methylbenzenesulfonyl, C(O)-C₁₋₄ alkyl, O-C₁₋₁₀ alkyl, O-benzyl, NH₂, NHR6 and N(R6)R7;
R6 and R7 are identical or different and have independently from each other and from R4 the same definition as R4.

4. Method according to claim 3, wherein
the salt of 4,4,4-trifluoro-3-oxobutyric acid is selected from the group consisting of N^{a}+, K⁺, NH₄⁺ and Ca²⁺ salt of 4,4,4-trifluoro-3-oxobutyric acid.

5. Method according to one or more of claims 1 to 4, wherein
base (BasS1) is selected from the group consisting of sodium hydride, potassium hydride, calcium hydride, sodium amide, lithium amide, LDA, N-lithium-2,2,6,6-tetramethylpiperidide, N-magnesium-2,2,6,6-tetramethylpiperidide, sodium methoxide, sodium ethoxide, sodium isopropoxide, sodium tert-butoxide, potassium tert-butoxide sodium carbonate, potassium carbonate, isopropylmagnesium bromide, isopropylmagnesium chloride, and mixtures thereof.

6. Method according to one or more of claims 1 to 5, wherein
R1 is selected from the group consisting of C₁₋₅ alkyl, C₃₋₆ cycloalkyl, C(O)-O-C₁₋₄ alkyl, CH=CH₂, benzyl and phenyl;
the C₁₋₁₀ alkyl of R1 is unsubstituted or substituted with 1, 2, 3, 4 or 5 identical of different substituents selected from the group consisting of halogen, OH, O-C(O)-CH₃, O-C₁₋₅ alkyl, S-C₁₋₅ alkyl, S(O)-C₁₋₅ alkyl, S(O₂)-C₁₋₅ alkyl, O-C₁₋₄ alkylen-O-C₁₋₄ alkyl, C₃₋₆ cycloalkyl and 1,2,4-triazolyl;
the benzyl and the phenyl of R1 are independently from each other unsubstituted or substituted with 1, 2, 3, 4 or 5 identical of different substituents selected from the group consisting of halogen, C₁₋₂ alkoxy, NO₂ and CN.

7. Method according to one or more of claims 1 to 6, wherein
Y is selected from the group consisting of OH, methoxy and ethoxy.

8. Method according to one or more of claims 1 to 7, wherein
R2 is selected from the group consisting of methyl and ethyl.

9. Method according to one or more of claims 1 to 8, wherein
R3 is selected from the group consisting of hydrogen, C₁₋₆ alkyl, benzyl, phenyl, pivaloyl, methanesulfonyl, benzenesulfonyl, 4-methylbenzenesulfonyl, methoxycarbonyl, ethoxycarbonyl, O-C₁₋₄ alkyl, O-benzyl, NH₂ and dimethylamino.

10. Method according to one or more of claims 1 to 9, wherein
reaction (ReacS1) is done in a solvent (SolvS1);
solvent (SolvS1) is selected from the group consisting of THF, 2-methyl-THF, 3-methyl-THF, DMF, DMA, acetonitrile, propionitrile, DMSO, 1,2-dimethoxyethane, methoxycyclopentane, diethyleneglycoldimethylether, diethyleneglycoldiethylether, benzene, toluene, anisole, and mixtures thereof.

11. Method for the preparation of compound of formula (I); 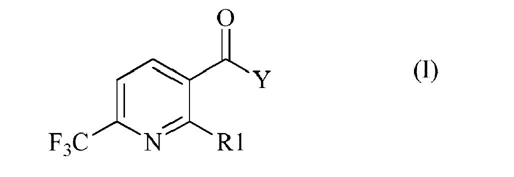 the method comprises the step (StepS1) as defined in claim 1 and a step (StepS2);
step (StepS2) comprises a reaction (ReacS2);
reaction (ReacS2) is a reaction of the compound of formula (II) prepared in step (StepS1) with a compound (AMS);
with compound of formula (II), R1 and Y as defined in claim 1;
compound (AMS) is selected from the group consisting of NH₃, sulfamic acid, urea, NH₂-C(O)-C₁₋₄ alkyl, ammonium carbonate, ammonium bicarbonate, ammonium salts of C₁₋₄ carboxylic acids, ammonium chloride and mixtures thereof.

12. Method according to claim 11, wherein
compound (AMS) is selected from the group consisting of NH₃, formamide, ammonium carbonate, ammonium bicarbonate, ammonium formate, ammonium acetate, ammonium chloride and mixtures thereof.

13. Method according to claim 11 or 12, wherein
reaction (ReacS2) is done in a solvent (SolvS2);
solvent (SolveS2) is selected from the group of C₁₋₆ alkanol, acetonitrile, propionitrile, C₁₋₄ carboxylic acid, benzene, toluene, xylene, chlorobenzene, anisole, pyridine, 2-methylpyridine, 3-methylpyridine, 4-methylpyridine, 2-methyl-5-ethylpyridine, DMF, DMA, DMSO, water, ethyl acetate, acetone, methylethylketone, THF, 2-methyl-THF, 3-methyl-THF, 1,2-dimethoxyethane, diethyleneglycoldimethylether and mixtures thereof.

14. Use of compound of formula (II), with compound of formula (II) having been prepared according to a method of one or more of claims 1 to 10, or of compound of formula (I), with compound of formula (I) having been prepared according to a method of one or more of claims 11 to 13, for the production of pharmaceutical, chemical or agro-chemical products.
